# EUROPEAN PATENT SPECIFICATION

(11) **EP 2 303 232 B1**
(45) Date of publication and mention of the grant of the patent: **02.08.2017**
(21) Application number: 09753848.2
(22) Date of filing: 25.05.2009
(51) Int. Cl.: A61K 9/14, A61K 31/137

(54) **Micronisable form of salmeterol xinafoate**
Mikronisierbare Form von Salmeterolxinafoat
Formule micronisable de xinafoate de salmétérol

(30) Priority: 26.05.2008 EP 08156932; 30.05.2008 US 57816 P
(43) Date of publication of application: 06.04.2011
(73) Proprietor: INKE, S.A., 08755 Bastellbisball - Barcelona (ES)
(72) Inventor: DALMASES BARJOAN, Pere, E-08980 Sant Feliu de Llobregat - Barcelona (ES); HUGUET CLOTET, Juan, E-08970 Sant Joan Despí - Barcelona (ES)
(74) Representative: ABG Patentes, S.L.
(86) International application number: PCT/EP2009/056285
(87) International publication number: WO 2009/144193

(56) References cited:
- EP-A- 0 639 176
- EP-A- 0 706 507
- GB-A- 2 140 800
- MURNANE D ET AL: "Comparison of salmeterol xinafoate microparticle production by conventional and novel antisolvent crystallization" EUROPEAN JOURNAL OF PHARMACEUTICS AND BIOPHARMACEUTICS, ELSEVIER SCIENCE PUBLISHERS B.V., AMSTERDAM, NL, vol. 69, no. 1, 1 May 2008 (2008-05-01), pages 94-105, XP022589602 ISSN: 0939-6411 [retrieved on 2008-04-05]

## Description

### FIELD OF THE INVENTION

The present invention relates to a novel micronisable form of salmeterol xinafoate, the process for its production and its use in the preparation of micronised salmeterol xinafoate.

### BACKGROUND

The phenethanolamine salmeterol xinafoate (I) is a long-active beta 2 adrenoreceptor agonist used in the treatment of bronchial asthma. Salmeterol xinafoate was described in GB 2140800 A together with its physiologically acceptable salts, specifically, its xinafoate salt. The drug is delivered by inhalation by means of either a metered-dose aerosol inhaler or a dry powder delivery device.

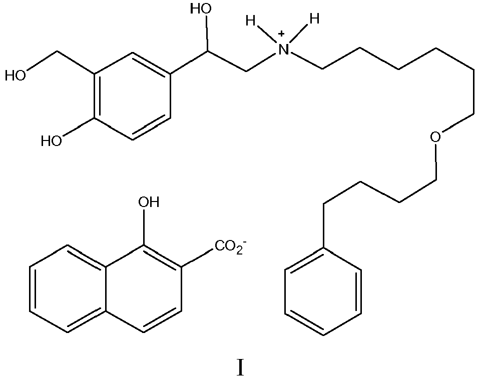

Before salmeterol xinafoate is formulated in the delivery device the drug substance is micronised to a size range about 2-5 µm to enable a suitable respirable fraction of drug particles to be delivered to the bronchial region of the lung. Micronisation is achieved using a fluid energy mill in which the crystalline powder is driven by air pressure into a cyclone.

Salmeterol xinafoate drug substance generated by a conventional crystallisation process has very poor powder flow properties, making it unsuitable for size reduction processes such as micronisation.

European patent EP 0 639 176 B1 provides a fast cooling crystallisation process which produces spherical agglomerates of small crystals which flow well and break up readily during micronisation.

European patent EP 0706507 B1 relates to a very rapid crystallization process using supercritical carbon dioxide which avoids micronisation of the final product. It also relates to salmeterol xinafoate in an easily fluidised crystalline form with a dynamic bulk density of less than 0.1 g.cm⁻³. According to the description, conventionally crystallised salmeterol xinafoate, when studied by differential scanning calorimetry (DSC), shows a transition between two forms (hereinafter "polymorph I" and "polymorph II") occurring between 120 and 140°C.

Henry H. Y. Tong et al in Pharmaceutical Research, 18, 6, 2001 describes the preparation of high pure polymorphs I and II of salmeterol xinafoate produced in a controlled one-step operation by the SEDS (Solution Enhanced Dispersion by Supercritical Fluids) technology.

However, some of the above-mentioned methods present several drawbacks derived from the use of supercritical fluids or mixtures of different boiling point solvents, which makes these procedures economically disadvantageous.

Therefore, it would be highly desirable to develop easy and economic methods to produce micronized salmeterol with good flowability properties, which overcome the drawbacks of the methods of the state of the art.

### BRIEF DESCRIPTION OF THE INVENTION

The authors of the present invention have surprisingly found a new micronisable form of salmeterol xinafoate polymorph I which shows good stability and appropriate physico-mechanical properties for its manipulation on industrial scale and final micronisation.

The novel micronisable form of salmeterol xinafoate is characterized for having a mean particle size lower than conventional crystallized micronisable salmeterol xinafoate, which facilitates the final micronisation of the product. Moreover, the inventors have found a process for preparing this novel micronisable salmeterol xinafoate which uses conventional crystallization methodologies and which does not require any especial equipment.

Thus, a first aspect of the invention provides a micronisable salmeterol xinafoate polymorph I, characterized by a mean particle size between 5 and 15 µm and a bulk density between 0.1 to 0.2 g/mL.

A second aspect of the invention relates to a process for the preparation of the micronisable form of salmeterol xinafoate polymorph I of the invention, said process comprising:
a) seeding a solution of salmeterol xinafoate in an organic solvent with salmeterol xinafoate polymorph I at a temperature approximately between 40 and 50°C;
b) subjecting the seeded solution of step a) to a stepwise profile cooling process. comprising between 2 and 5 steps, wherein the stepwise profile cooling process starts at a temperature between 40°C and 50°C and ends at a temperature between 5°C and -10°C and wherein the temperature is kept constant in each step during a time between 30 minutes and 2 hours.

This process has the advantage of being economical and easily industrialized.

Finally, another aspect of the invention is directed to the use of such novel micronisable salmeterol xinafoate in the preparation of micronised salmeterol xinafoate.

### DETAILED DESCRIPTION OF THE INVENTION

As mentioned above, it is an object of the present invention to provide a novel micronisable form of salmeterol xinafoate polymorph I, characterized by a mean particle size between 5 and 15 µm and a bulk density between 0.1 to 0.2 g/mL.

As defined in the present patent application the extremes of the ranges of the parameters are considered within the scope of the invention.

By the term "micronisable form" it is understood a form which is easily broken down under micronising conditions, for example in a fluid-energy mill, to particles of a size suitable for use in a pharmaceutical dosage form to be delivered by inhalation or insufflation.

A feature derived from being micronisable is that the novel form is also free-flowing. This means that the form flows freely into a powder mill, for example a fluid energy powder mill, to allow its efficient particle size reduction by micronisation on an industrial scale. The physical characteristics of a material that determine its flow characteristics include its bulk density, cohesivity, specific surface area, particle size and shape and uniformity with respect to the particle size.

As defined by the European Pharmacopoeia 6.0, Chapter 2.9.34:
- "Bulk density" of a powder is the ratio of the mass of an untapped powder sample to its volume, including the contribution of the interparticulate void volume. Hence, the bulk density depends on both, the density of powder particles and the spatial arrangement of particles in the powder bed. The bulk density is expressed in grams per milliliter or grams per cubic centimeter.
- "Tapped density" is a density attained after mechanically tapping a receptacle containing the powder sample.

The bulk density is determined by measuring the volume of a known mass of powder that has been passed through a screen, into a graduated cylinder.

The tapped density is achieved by mechanically tapping a measuring cylinder containing a powder sample. After observing the initial volume, the cylinder is mechanically tapped, and volume readings are taken until further volume change is observed.

The bulk density and the tapped density as defined in the invention are measured following respectively Method 1 and Method 2 described in European Pharmacopoeia Chapter 6.0, 2.9.34.

The novel micronisable form of the invention exhibits a bulk density between 0.1 and 0.2 g/mL, preferably between 0.12 and 0.17 g/mL.

When compared against conventionally crystallized micronisable salmeterol xinafoate, the micronisable form of the present invention exhibits a lower bulk density, which facilitates the preparation of micronized salmeterol xinafoate with well-balanced physico-mechanical properties in order to be administered by inhalation or insufflation.

Cohesiveness is a measure of the inter-particle forces (e.g. van der Waals forces). As particle size decreases, the inter-particle forces become stronger leading to an increase in cohesion. The increase in cohesion plays a dominant role in flow dynamics as it directly impacts the bulk flowabilibty of solid materials.

Specific surface area in the sense of the present invention means the BET-surface area, i.e. the surface area determined by nitrogen adsorption according to the method Brunauer, Emmet and Teller, S. Lowell and J.E. Shields, Powder Surface Area and Porosity, 1984, 2nd edition.

According to European Pharmacopoeia 6.0, chapter 2.9.36, compressibility is one of the standardized methods for characterizing powder flow.

The novel micronisable form of the invention has a mean particle size between 5 and 15 µm, preferably between 6 and 12 µm. This particle size is determined according to the monograph of European Pharmacopoeia 6.0, chapter 2.9.31. When compared against conventionally crystallized micronisable salmeterol xinafoate, the micronisable form of the present invention exhibits a lower mean particle size lower, which facilitates the final micronisation of the product.

A scanning electron micrograph (SEM) of some crystals of the novel micronisable salmeterol xinafoate shows that they are stable agglomerates of platelike crystals.

The present novel micronisable form may be prepared by any suitable method. However, in a particular embodiment, there is provided a process for the preparation of the micronisable form of salmeterol xinafoate polymorph I of the invention, said process comprising:
a) seeding a solution of salmeterol xinafoate in an organic solvent with salmeterol xinafoate polymorph I at a temperature approximately between 40 and 50°C;
b) subjecting the seeded solution of step a) to a stepwise profile cooling process.

The authors of the present invention have found that the use of relatively low seeding temperatures, around 40-50°C, allows a subsequent rapid formation of the crystals of salmeterol xinafoate with an adequate mean particle size to be micronized. When the seeding temperature used is higher than about 55°C, the mean particle size of the resulting crystals reach over 20 µm, which makes difficult the final micronisation process.

In a preferred embodiment, the concentration of salmeterol xinafoate in the organic solvent solution before seeding is between 5 and 15 g/100 g solvent, more preferably between 8 and 10 g/100 g solvent.

Salmeterol xinafoate used as starting material for the purposes of this invention is prepared according to the process described in European patent EP 1132373 B1.

The term "stepwise profile cooling process" refers to a cooling process comprising two or more steps, wherein each step is carried out at a lower temperature than the previous one. Each step comprises a first period during which temperature is decreased followed by a second period during which temperature is kept constant.

In a particular embodiment, the cooling process starts, after seeding, at a temperature between 40°C and 50°C, more preferably between 43 and 47°C and ends at a temperature between 5°C and -10°C, more preferably between 0 and -5°C. In a still more preferably embodiment, the cooling process starts at 45°C and ends at -5°C. This process comprises between 2 and 5 steps and the suitable time to keep the temperature constant in each step is between 30 min and 2 hours.

In a particular embodiment, the process of the invention further comprises a previous step wherein salmeterol xinafoate is dissolved in the organic solvent at its boiling point and subsequently, said solution is subjected to a cooling step to reach a temperature between 40 and 50°C to carry out step a) of the process of the invention.

In a preferred embodiment, the organic solvent is an aliphatic ketone. Illustrative aliphatic ketones for use in the present invention include acetone, methyl isobutyl ketone, ethyl butyl ketone, dihexyl ketone, methyl ethyl ketone, diethyl ketone, diisobutyl ketone and mixtures thereof. Preferably, methyl ethyl ketone is used.

Unexpectedly, the process developed by the inventors provides polymorph I of salmeterol xinafoate with a mean particle between 5 and 15 µm, preferably between 6 and 12 µm and a bulk density between 0.1 to 0.2 g/mL, which is stable and suitable for manipulation and micronisation on an industrial scale.

In a third aspect, the invention is directed to the use of the novel micronisable salmeterol xinafoate of the invention in the preparation of micronised salmeterol xinafoate. Preferably, the micronisable form of salmeterol xinafoate is micronised until the collected material has a particle size that is suitable for pharmaceutical dosage forms to be delivered by inhalation or insufflation. A suitable particle size for this use is less 5 µm, preferably less than 3 µm.

The micronization process may be carried out by any conventional micronizing techniques known by a skilled person, for example with a Trost or jet mill.

The following example is displayed to illustrate the micronisable form of the present invention and the process for its preparation. They do not intend to limit in any way the scope of the invention defined in the present description.

### EXAMPLES

### Example 1:

A suspension of 23 g of salmeterol xinafoate in 310 mL of methyl ethyl ketone is warmed to 70 °C under nitrogen. The solution obtained is cooled to 45 °C and seeded with 0.3 g of salmeterol xinafoate polimorph 1. The solution/suspension is cooled further to 35 - 40 °C and stirring is kept on for 30 min at that temperature. The suspension is cooled down slowly to room temperature (20 - 25 °C) and stirred for 1 hour. The suspension is cooled down again to 0 °C and stirred for at least 1 hour at that temperature. The slurry is filtered under nitrogen pressure, washed with 40 mL of methyl ethyl ketone pre-cooled at 0 °C and the solid is dried under vacuum at 30 °C. 21 g (91.3 %) of salmeterol xinafoate polimorph I are obtained with a mean particle size of 11.5 µm.

The physical properties of the novel micronisable salmeterol xinafoate obtained are:

| **Physical Property** | **Salmeterol xinafoate** |
|---|---|
| Bulk density (g·ml⁻¹) | 0.136 |
| Tapped density (g·ml⁻¹) | 0.256 |
| Compressibility (%) | 47.94 |
| BET Surface area (m²·g⁻¹) | 0.82 |
| Mean particle size (µm) | 11.5 |
| Cohesiveness | 74.8 |

## Claims

1. A process for preparing the micronisable salmeterol xinafoate polymorph I, wherein the micronisable salmeterol xinafoate polymorph I is **characterized by** a mean particle size between 5 and 15 µm and a bulk density between 0.1 and 0.2 g/mL, said process comprising:
a. seeding a solution of salmeterol xinafoate in an organic solvent with salmeterol xinafoate polymorph I at a temperature approximately between 40 and 50°C;
b. subjecting the seeded solution of step a) to a stepwise profile cooling process comprising between 2 and 5 steps, wherein the stepwise profile cooling process starts at a temperature between 40°C and 50°C and ends at a temperature between 5°C and -10°C and wherein the temperature is kept constant in each step during a time between 30 minutes and 2 hours.

2. The process according to claim 1 wherein the concentration of salmeterol xinafoate in the organic solvent solution before seeding is between 5 and 15 g/100 g solvent, more preferably between 8 and 10 g/100 g solvent.

3. The process according to claim 2 wherein the stepwise profile cooling process starts at a temperature between 43°C and 47°C and ends at a temperature between 0°C and -5°C.

4. The process according to any one of claims 1 to 3 which further comprises a previous step wherein salmeterol xinafoate is dissolved in the organic solvent at its boiling point and subsequently, said solution is subjected to a cooling step to reach a temperature between 40 and 50°C.

5. The process according to any one of claims 1 to 4 wherein the organic solvent is an aliphatic ketone selected from the group consisting of acetone, methyl isobutyl ketone, ethyl butyl ketone, dihexyl ketone, methyl ethyl ketone, diethyl ketone, diisobutyl ketone and mixtures thereof.

## Patentansprüche

1. Verfahren zur Herstellung des mikronisierbaren Salmeterolxinafoat-Polymorphs I, wobei das mikronisierbare Salmeterolxinafoat-Polymorph I durch eine mittlere Teilchengröße zwischen 5 und 15 µm und eine Schüttdichte zwischen 0,1 und 0,2 g/ml gekennzeichnet ist, wobei das Verfahren umfasst:
a. Impfen einer Lösung von Salmeterolxinafoat in einem organischen Lösungsmittel mit Salmeterolxinafoat-Polymorph I bei einer Temperatur ungefähr zwischen 40 und 50 °C;
b. Durchführen eines stufenweisen Profilkühlungsverfahrens, das zwischen 2 und 5 Stufen umfasst, mit der geimpften Lösung aus Schritt a), wobei das stufenweise Profilkühlungsverfahren bei einer Temperatur zwischen 40 °C und 50 °C beginnt und bei einer Temperatur zwischen 5 °C und -10 °C endet und wobei die Temperatur bei jeder Stufe während einer Zeit zwischen 30 Minuten und 2 Stunden konstant gehalten wird.

2. Verfahren gemäß Anspruch 1, wobei die Konzentration von Salmeterolxinafoat in der Lösung in dem organischen Lösungsmittel vor dem Impfen zwischen 5 und 15 g/100 g Lösungsmittel, besonders bevorzugt zwischen 8 und 10 g/100 g Lösungsmittel, beträgt.

3. Verfahren gemäß Anspruch 2, wobei das stufenweise Profilkühlungsverfahren bei einer Temperatur zwischen 43 °C und 47 °C beginnt und bei einer Temperatur zwischen 0 °C und -5 °C endet.

4. Verfahren gemäß einem der Ansprüche 1 bis 3, das weiterhin einen vorangehenden Schritt umfasst, bei dem Salmeterolxinafoat in dem organischen Lösungsmittel an dessen Siedepunkt gelöst wird und die Lösung anschließend einem Kühlungsschritt unterzogen wird, um eine Temperatur zwischen 40 und 50 °C zu erreichen.

5. Verfahren gemäß einem der Ansprüche 1 bis 4, wobei das organische Lösungsmittel ein aliphatisches Keton ist, das aus der Gruppe ausgewählt ist, die aus Aceton, Methylisobutylketon, Ethylbutylketon, Dihexylketon, Methylethylketon, Diethylketon, Diisobutylketon und Gemischen davon besteht.

## Revendications

1. Un procédé de préparation du polymorphe I de xinafoate de salmétérol micronisable, où le polymorphe I de xinafoate de salmétérol micronisable est **caractérisé par** une taille moyenne de particules entre 5 et 15 µm et une masse volumique apparente entre 0,1 et 0,2 g/mL, ledit procédé comprenant :
a. ensemencer une solution de xinafoate de salmétérol dans un solvant organique avec du polymorphe I de xinafoate de salmétérol à une température entre 40 °C et 50 °C environ ;
b. soumettre la solution ensemencée de l'étape a) à un procédé de rafraîchissement par étapes comprenant entre 2 et 5 étapes, où le procédé de rafraîchissement par étapes commence à une température entre 40 °C et 50 °C et se termine à une température entre 5 °C et -10 °C et où la température est maintenue constante dans chaque étape pendant une durée entre 30 minutes et 2 heures.

2. Le procédé selon la revendication 1 où la concentration de xinafoate de salmétérol dans la solution de solvant organique avant l'ensemencement est comprise entre 5 et 15 g pour 100 g de solvant, de préférence entre 8 et 10 g pour 100 g de solvant.

3. Le procédé selon la revendication 2, où le procédé de rafraîchissement par étapes commence à une température entre 43 °C et 47 °C et se termine à une température entre 0 °C et -5 °C.

4. Le procédé selon l'une quelconque des revendications 1 à 3, qui comprend en outre une étape précédente dans laquelle du xinafoate de salmétérol est dissous dans le solvant organique à son point d'ébullition, puis ladite solution est soumise à une étape de rafraîchissement afin d'atteindre une température entre 40 °C et 50 °C.

5. Le procédé selon l'une quelconque des revendications 1 à 4, où le solvant organique est un cétone aliphatique sélectionné parmi le groupe constitué d'acétone, méthylisobutylcétone, éthylbutylcétone, dihexylcétone, méthyléthylcétone, diéthylcétone, diisobutylcétone et de mélanges de ceux-ci.
